# EUROPÄISCHE PATENTSCHRIFT

(11) **EP 3 215 598 B1**
(45) Veröffentlichungstag und Bekanntmachung des Hinweises auf die Patenterteilung: **05.01.2022**
(21) Anmeldenummer: 15800731.0
(22) Anmeldetag: 05.11.2015
(51) Int. Cl.: C12M 1/107, C12M 1/06, C12M 1/34, C12M 1/36, B01F 7/00

(54) **VERFAHREN ZUM BETREIBEN EINER RÜHREINRICHTUNG UND EINES FERMENTERS**
METHOD FOR OPERATING A STIRRING DEVICE AND A FERMENTER
PROCÉDÉ PERMETTANT DE FAIRE FONCTIONNER UN AGITATEUR ET UN FERMENTEUR

(30) Priorität: 07.11.2014 DE 102014116239
(43) Veröffentlichungstag der Anmeldung: 13.09.2017
(73) Patentinhaber: UTS Biogastechnik GmbH, 85399 Hallbergmoos (DE)
(72) Erfinder: CZWALUK, Andreas, 49377 Vechta (DE)
(74) Vertreter: BSB Patentanwälte Schütte & Engelen Partnerschaft mbB
(86) Internationale Anmeldenummer: PCT/EP2015/075818
(87) Internationale Veröffentlichungsnummer: WO 2016/071447

(56) Entgegenhaltungen:
- EP-A1- 0 516 895
- EP-A1- 1 884 561
- EP-A1- 2 559 750
- WO-A1-2011/121022
- WO-A1-2011/121024
- WO-A2-2014/068016
- CN-A- 102 851 408
- DE-A1- 10 056 338
- DE-A1-102010 050 863
- DE-U1-202007 002 835

## Beschreibung

Die vorliegende Erfindung betrifft ein Verfahren zum Betreiben einer Rühreinrichtung und eines Fermenters zum Herstellen von Biogas mit einem wenigstens teilweise mit Substrat gefüllten Fermenter. Dabei ist in dem Fermenter wenigstens eine über eine Steuereinrichtung gesteuerte Rühreinrichtung angeordnet.

Im Stand der Technik sind verschiedene Verfahren zum Herstellen von Biogas bekannt geworden, wobei regelmäßig wenigstens ein Fermenter vorgesehen ist, der ein Substrat aufweist, welchem kontinuierlich oder periodisch Material zugeführt wird. Oberhalb des Substratspiegels sammelt sich Biogas an, welches ebenfalls kontinuierlich oder periodisch abgeführt wird, um es in ein Gasnetz einzuspeisen oder aber direkt an der Biogasanlage daraus Strom zu erzeugen.

Die WO 2011/121024 A1 offenbart ein Verfahren zur Herstellung von Biogas aus organischen Stoffen, wobei einem Behälter Substrat mittels eines Aufgabesystems zugeführt wird und wobei in dem Behälter zwei Rührwerke angeordnet sind, deren Propeller über Antriebe in Rotation versetzt werden. Die Propeller erzeugen zumeist horizontale Strömungen in dem Behälterinhalt und die Durchmesser der Propeller und deren Geometrie und die Position der Propeller ist so gewählt, dass eine gemeinsame Mischzone des Gärsubstrates in dem Behälter erzeugbar ist. Es werden Messdaten zur Ermittlung der mittleren Geschwindigkeit und der mittleren Viskosität des Gärsubstrates in der Mischzone erfasst und die Messdaten werden an eine Regeleinheit weitergeleitet. Durch die Regeleinheit werden Stellgrößen variiert, welche den Leistungseintrag der Rührwerke in die Mischzone und die Zusammensetzung und das Fließverhalten des Behälterinhalts verändern.

Mit der WO 2011/121022 ist ein Verfahren und eine Vorrichtung zur Herstellung von Biogas aus organischen Stoffen bekannt geworden, wobei einem Behälter Substrat mittels eines Aufgabesystems zugeführt wird und in dem Behälter ein Rührwerk zum Vermischen des Behälterinhalts vorgesehen ist. Bei diesem bekannten Verfahren wird die Bildung von Kavernen im propellernahen Bereich ermittelt und in Abhängigkeit davon wird ein betriebsspezifischer Sollwert vorgegeben. Der Vorteil dieses Verfahrens liegt darin, dass im propellernahen Bereich, also direkt benachbart zu den Rührflügeln, die Bildung von Kavernen berücksichtigt wird. Durch diese Regelung kann Kavitation zuverlässig vermieden werden. Nachteilig daran ist aber, dass das im Behälter vorhandene Substrat und dessen charakteristische Eigenschaften nicht genügend berücksichtigt werden.

Es ist deshalb die Aufgabe der vorliegenden Erfindung, ein Verfahren zum Herstellen von Biogas zur Verfügung zu stellen, womit die Eigenschaften des in dem Fermenter vorhandenen Substrates besser berücksichtigt werden.

Diese Aufgabe wird gelöst durch ein Verfahren mit den Merkmalen des Anspruchs 1. Bevorzugte Weiterbildungen der Erfindung sind Gegenstand der Unteransprüche. Weitere Vorteile und Merkmale der vorliegenden Erfindung ergeben sich aus dem Ausführungsbeispiel.

Das erfindungsgemäße Verfahren dient zum Herstellen von Biogas mit wenigstens einem wenigstens teilweise mit Substrat gefüllten Fermenter. In dem Fermenter ist wenigstens eine über eine Steuereinrichtung gesteuerte Rühreinrichtung angeordnet.

Erfindungsgemäß sind wenigstens die folgenden Verfahrensschritte in der aufgeführten Reihenfolge vorgesehen:
a) in der Steuereinrichtung wird eine Soll-Lastkurve hinterlegt, die insbesondere charakteristisch für das in dem Fermenter vorhandene Substrat ist,oder die Steuerung greift auf eine hinterlegte Soll-Lastkurve zu
b) die Steuereinrichtung gibt eine Soll-Drehzahl vor.
c) die Steuereinrichtung betreibt die Rühreinrichtung mit einer Ist-Drehzahl, die der vorgegebenen Soll-Drehzahl entspricht.
d) die Steuereinrichtung erfasst einen Ist-Messwert, der für das Drehmoment der Rühreinrichtung bei der Ist-Drehzahl charakteristisch ist.
e) die Steuereinrichtung leitet aus dem Ist-Messwert einen Ist-Kennwert für das aufgebrachte Drehmoment der Rühreinrichtung ab.
f) die Steuereinrichtung vergleicht den abgeleiteten Ist-Kennwert mit dem sich aus der Soll-Lastkurve bei der vorgegebenen Soll-Drehzahl ergebenden Soll-Kennwert für das Substrat.
g) die Steuereinrichtung steuert die Rühreinrichtung in Abhängigkeit von dem Ergebnis des Vergleiches.

Das erfindungsgemäße Verfahren hat viele Vorteile. Durch das erfindungsgemäße Verfahren wird es ermöglicht, eine Soll-Lastkurve für das derzeit eingesetzte Substrat in der Steuereinrichtung zu hinterlegen und den Betrieb der Rühreinrichtung in Abhängigkeit von dieser Soll-Lastkurve zu steuern. Weicht das Substrat in seinen Eigenschaften zu gewissen Zeitpunkten von der Soll-Kurve ab, wird durch die Steuereinrichtung gesteuert die Rühreinrichtung entsprechend betrieben, um das Substrat zum Beispiel wieder wie gewünscht zu homogenisieren.

Unter Substrat wird im Sinne der vorliegenden Anmeldung ein Substrat aus organischen Stoffen bzw. ein Gärsubstrat verstanden. Den organischen Stoffen können organische oder anorganische Zusatzstoffe beigemengt werden, um die gewünschte Reaktion zu unterstützen.

Zu Beginn des Verfahrens oder ursprünglich wird der Verfahrensschritt a) durchgeführt und es wird eine Soll-Lastkurve für ein Substrat oder eine Mischung in der Steuereinrichtung hinterlegt, also dort oder woanders gespeichert. Ein solcher Verfahrensschritt kann auch regelmäßig oder periodisch im Betrieb erfolgen, wenn sich beispielsweise das Substrat oder dessen Zusammensetzung ändert.

Der Ist-Messwert ist charakteristisch für das Drehmoment bei der Ist-Drehzahl. Das bedeutet, dass aus dem Ist-Messwert ein charakteristischer Wert für das aktuelle Drehmoment abgeleitet werden kann. Beispielsweise kann als Messwert direkt ein Maß für das Drehmoment erfasst werden. Es ist aber auch möglich, dass beispielsweise die von der Rühreinrichtung benötigte elektrische Leistung als Messwert eingesetzt wird. Über die bekannten physikalischen Formeln kann aus der elektrischen Leistung und der bekannten Ist-Drehzahl auf das Drehmoment zurückgeschlossen werden. Dabei können auftretende Verluste herausgerechnet werden. So ist beispielsweise der Wirkungsgrad eines elektrischen Motors oder eines hydraulischen Motors und der Wirkungsgrad der Umsetzung eines Getriebes eine gerätespezifische und gegebenenfalls leistungsabhängige Konstante. Derartige Konstanten oder Kennfelder können in der Steuereinrichtung hinterlegt werden, sodass beispielsweise aus der aktuell benötigten Ist-Leistung der Rühreinrichtung ausreichend genau auf das Ist-Drehmoment zurückgeschlossen werden kann.

In einer bevorzugten Weiterbildung ermittelt die Steuereinrichtung in einem Verfahrensschritt h), ob der Ist-Kennwert innerhalb eines vorgegebenen Toleranzbereichs um die Soll-Lastkurve bei der Soll-Drehzahl liegt. Dadurch kann das Ergebnis des Vergleichs in drei Bereiche aufgeteilt werden, nämlich entweder liegt der Ist-Kennwert unterhalb der Soll-Lastkurve und außerhalb des vorgegebenen Toleranzbereichs, oder der Ist-Kennwert liegt oberhalb der Soll-Lastkurve und außerhalb des vorgegebenen Toleranzbereichs, oder der Ist-Kennwert liegt innerhalb des vorgegebenen Toleranzbereichs. Dabei spielt es keine Rolle, ob ein Ist-Messwert genau auf der Soll-Lastkurve oder leicht daneben, aber innerhalb des vorgegebenen Toleranzbereichs liegt.

In allen Weiterbildungen ist es besonders bevorzugt, dass der Toleranzbereich drehzahlabhängig sein kann. Möglich ist es auch, dass der Toleranzbereich von dem Drehmoment oder von der aktuellen Leistung abhängt. Möglich ist es auch, dass der Toleranzbereich eine Konstante oder eine relative Abweichung von der Soll-Lastkurve definiert.

Vorzugsweise werden die folgenden Verfahrensschritte i1) bis i4) in einer Schleife gegebenenfalls wiederholt durchgeführt, falls der Ist-Kennwert unterhalb des Soll-Kennwerts und außerhalb des Toleranzbereichs liegt:
i1) die Ist-Drehzahl der Rühreinrichtung wird um ein vorbestimmtes Maß erhöht.
i2) es wird ein neuer Ist-Messwert bei der neuen Ist-Drehzahl erfasst.
i3) es wird aus dem neuen Ist-Messwert ein neuer Ist-Kennwert für das Drehmoment der Rühreinrichtung abgeleitet.
i4) die Schleife wird verlassen, wenn der abgeleitete neue Ist-Kennwert innerhalb des vorgegebenen Toleranzbereichs der Soll-Lastkurve bei der Soll-Drehzahl liegt.

In einer weiteren bevorzugten Weiterbildung werden entsprechend die folgenden Verfahrensschritte j1) bis j4) in einer Schleife durchgeführt, falls der Ist-Kennwert für das Drehmoment oberhalb des Soll-Kennwerts und außerhalb des Toleranzbereichs liegt:
j1) die Ist-Drehzahl der Rühreinrichtung wird um ein vorbestimmtes Maß verringert.
j2) es wird ein neuer Ist-Messwert bei der neuen Ist-Drehzahl erfasst.
j3) es wird aus dem neuen Ist-Messwert ein neuer Ist-Kennwert für das Drehmoment der Rühreinrichtungabgeleitet.
j4) die Schleife wird verlassen, wenn der abgeleitete neue Ist-Kennwert innerhalb des vorgegebenen Toleranzbereichs der Soll-Lastkurve bei der Soll-Drehzahl liegt.

Vorzugsweise ist die Größe des Toleranzbereiches abhängig von dem Soll-Kennwert (Drehmoment) der Soll-Lastkurve bei der Soll-Drehzahl. In einfachen und bevorzugten Ausgestaltungen definiert der Toleranzbereich eine prozentuale Abweichung. In bevorzugten Ausgestaltungen erlaubt der Toleranzbereich +/-33% Abweichung von dem Soll-Kennwert für das Drehmoment. In bevorzugten Ausgestaltungen definiert der Toleranzbereich eine Abweichung von bis zu +/-25% Abweichung von dem Soll-Kennwert. In Weiterbildungen kann der Toleranzbereich auf +/-20% oder insbesondere auch +/-10% Abweichung oder möglicherweise auch +/-5% Abweichung von dem Soll-Kennwert beschränkt werden. Regelmäßig ist eine noch weitere Einschränkung des Toleranzbereiches nicht vorgesehen. Dadurch werden z. B. Regelschwankungen vermieden. Insbesondere geht es primär nicht um eine Energieoptimierung und auch nicht um eine Konstanthaltung der eingebrachten Leistung, sondern darum, dass Rührergebnis zu optimieren. Es soll regelmäßig auch keine Nachregelung der eingebrachten Leistung durchgeführt werden, sondern eine Einstellung gewünschter Betriebspunkte, um eine optimale Durchmischung des Substrates zu gewährleisten.

Der Toleranzbereich ist vorzugsweise von der Soll-Drehzahl abhängig und nicht von der Ist-Drehzahl. Der Toleranzbereich kann prozentual oder stufenweise über der Drehzahl verändert werden.

Sollte in einem Schritt die Soll-Lastkurve und der Toleranzbereich vollständig überschritten werden, so kann entsprechend gegengesteuert werden. Falls also zunächst der Ist-Kennwert oberhalb des Soll-Kennwerts und außerhalb des Toleranzbereichs liegt und nach der Verringerung der Ist-Drehzahl um ein vorbestimmtes Maß nicht innerhalb des Toleranzbereichs liegt, sondern unterhalb der Soll-Lastkurve und außerhalb des Toleranzbereichs, so wird die Ist-Drehzahl im nächsten Verfahrensschritt beispielsweise um ein halbes vorbestimmtes Maß erhöht. Entsprechend umgekehrt kann vorgegangen werden, falls der Ist-Kennwert zunächst unterhalb des Soll-Kennwerts und außerhalb des Toleranzbereichs liegt und nach der Erhöhung der Ist-Drehzahl oberhalb des Soll-Kennwerts und außerhalb des Toleranzbereichs liegt.

In besonders bevorzugten Weiterbildungen wird die eingestellte Ist-Drehzahl für den Rest eines Rührzyklus oder eine vorbestimmte Zeitdauer beibehalten, wenn der Ist-Kennwert innerhalb des vorgegebenen Toleranzbereichs um die Soll-Lastkurve bei der vorgegebenen Soll-Drehzahl liegt. Dadurch wird dem System zur Homogenisierung Zeit gegeben. Zu häufige Regelungsschritte, die zu Instabilitäten führen könnten, werden vermieden.

Vorzugsweise wird am Ende eines Rührzyklus oder nach einer vorbestimmten Zeit die Rühreinrichtung abgeschaltet oder in der Leistung erheblich reduziert. Während eines sich anschließenden Ruhezyklus bleibt die Rühreinrichtung abgeschaltet oder wird mit erheblich verringerter Leistung weiter betrieben. Dadurch wird insgesamt erheblich Energie eingespart, da die Rühreinrichtung nicht kontinuierlich über den gesamten Betriebszeitraum angetrieben werden muss. In den meisten Fällen reicht eine periodische Umrührung völlig aus. Wird beispielsweise ein Rührzyklus von 10 Minuten oder von 1 Stunde und eine entsprechende Ruhepause von 5 Minuten, 10 Minuten, 20 Minuten bzw. eine halbe Stunde oder 1 Stunde oder 2 Stunden vorgesehen, so kann der insgesamt benötigte Energiebedarf um 30 %, 50 % oder mehr reduziert werden. Für das Energieeinsparen ist es letztlich fast egal, ob die Rühreinrichtung mit beispielsweise 10 % der Leistung weiter betrieben wird oder komplett abgeschaltet wird.

Vorzugsweise schließt sich an einen Ruhezyklus ein neuer Rührzyklus an. Besonders bevorzugt wechseln sich periodisch nacheinander ein Rührzyklus und ein Ruhezyklus ab.

Beim Start eines neuen Rührzyklus werden vorzugsweise die oben angeführten Verfahrensschritte b), c), d), e), f), g) und h) vorgenommen. Das bedeutet, dass die Steuereinrichtung zunächst eine Soll-Drehzahl vorgibt und dass die Steuereinrichtung anschließend die Rühreinrichtung mit einer Ist-Drehzahl betreibt, die der vorgegebenen Soll-Drehzahl entspricht. Die Steuereinrichtung erfasst einen Ist-Messwert, der für das Drehmoment der Rühreinrichtung bei der Ist-Drehzahl charakteristisch ist. Die Steuereinrichtung leitet aus dem Ist-Messwert einen Ist-Kennwert für das aufgebrachte Drehmoment der Rühreinrichtung ab. Weiterhin steuert die Steuereinrichtung die Rühreinrichtung in Abhängigkeit von dem Ergebnis des Vergleichs. Schließlich ermittelt die Steuereinrichtung, ob der Ist-Kennwert innerhalb eines vorgegebenen Toleranzbereichs um die Soll-Lastkurve bei der Soll-Drehzahl liegt.

Eine neue Soll-Lastkurve muss nicht vorgegeben werden. Es ist aber möglich, die Soll-Lastkurve bei Bedarf oder periodisch anzupassen oder neu vorzugeben.

Vorzugsweise werden in Abhängigkeit von von den erzielten Ergebnissen die oben angeführten Verfahrensschritte die i1) bis i4) und/oder die Verfahrensschritte j1) bis j4) gegebenenfalls wiederholt durchgeführt. Das bedeutet, dass die Ist-Drehzahl der Rühreinrichtung um ein vorbestimmtes Maß erhöht wird, falls der Ist-Kennwert unterhalb des Soll-Kennwerts und außerhalb des Toleranzbereichs liegt. In entsprechender Weise wird die Ist-Drehzahl der Rühreinrichtung um ein vorbestimmtes Maß verringert, wenn der Ist-Kennwert oberhalb des Soll-Kennwerts und außerhalb des Toleranzbereichs liegt. Nach der Erhöhung bzw. Verringerung der Ist-Drehzahl wird bei der neuen Ist-Drehzahl ein neuer Ist-Messwert erfasst und daraus ein neuer Ist-Kennwert abgeleitet. Die Schleife wird durchgelaufen, bis der Ist-Kennwert innerhalb des vorgegebenen Toleranzbereichs um die Soll-Lastkurve liegt oder bis die Schleife gegebenenfalls nach einer vorbestimmten Anzahl von Schleifendurchgängen abgebrochen wird.

Die vorgegebene Soll-Drehzahl hängt vorzugsweise von dem Substrat und/oder dessen Zusammensetzung und/oder der gewünschten Biogasproduktion ab.

Vorzugsweise wird in jedem Rührzyklus eine Ist-Drehzahl in Abhängigkeit von der Soll-Lastkurve eingestellt. Die Einstellung der Ist-Drehzahl kann iterativ erfolgen, bis die Ist-Drehzahl innerhalb des vorgegebenen Toleranzbereichs um die Soll-Lastkurve bei der Soll-Drehzahl liegt. Nach der Einstellung der Ist-Drehzahl wird die Ist-Drehzahl für den Rest des Rührzyklus beibehalten. Dabei wird ein erster Zähler inkrementiert, wenn die Ist-Drehzahl größer als die vorgegebene Soll-Drehzahl ist und/oder es wird ein zweiter Zähler inkrementiert, wenn die Ist-Drehzahl kleiner als die vorgegebene Soll-Drehzahl ist. Dadurch wird ein einfaches Maß dafür ermittelt, ob die Soll-Lastkurve jedes Mal direkt erreicht wird, oder ob die Ist-Drehzahl jeweils verändert werden muss. Außerdem wird die Anzahl der Veränderungen registriert, sodass diese auswertbar ist.

Demzufolge ist es bevorzugt, die Zuführmenge von dem Substrat und/oder die Zusammensetzung des zugeführten Substrats und/oder die Art und den Takt des Betriebs der Rühreinrichtung zu verändern und/oder Zusatzstoffe zuzuführen, wenn der erste und/oder der zweite Zähler eine vorgegebene Schwelle überschreitet.

Beispielsweise kann eine zu häufige Abweichung des Ist-Kennwerts von dem Soll-Kennwert auf eine nicht ausreichende Durchmischung oder eine nicht korrekte Zusammensetzung des Substrats zurückzuführen sein.

Die vorgegebene Schwelle hängt insbesondere auch von der Anzahl der Rührzyklen ab. Möglich und bevorzugt ist es auch, den ersten oder den zweiten Zähler zurückzusetzen, wenn der jeweils andere Zähler erhöht wird. Dann wird bei einem Pendeln des Ist-Kennwerts um die Soll-Lastkurve von Rührzyklus zu Rührzyklus keine weitere Aktion nötig. Gegebenenfalls können langfristig Auswertungen gezogen werden, um das System insgesamt zu verbessern.

Vorzugsweise wird bei einer vorbestimmten Anzahl von direkt aufeinanderfolgenden Änderungen des ersten und/oder des zweiten Zählers die Zuführmenge von Substrat von außen und/oder die Zusammensetzung des zugeführten Substrats verändert und/oder es werden Zusatzstoffe zugeführt.

In allen Ausgestaltungen ist es bevorzugt, dass als Ist-Messwert ein Maß für die elektrische Leistung der Rühreinrichtung oder für ein Drehmoment der Rühreinrichtung gemessen wird. Als Ist-Messwert für die elektrische Leistung kann die elektrische Leistung des Rührgeräts insgesamt verwendet werden. Zur Bestimmung des Drehmoments kann auch ein Dehnungsmessstreifen eingesetzt werden.

Vorzugsweise betreibt die Steuereinrichtung die Rühreinrichtung insbesondere bei Bedarf, in regelmäßigen Abständen oder z. B. bei einem Neustart nach einer Wartung oder z. B. nach Zufuhr einer Charge an Substrat mit einer Mehrzahl von unterschiedlichen Drehzahlen und erfasst jeweils ein Maß für die erforderliche Leistung der Rühreinrichtung bei den unterschiedlichen Drehzahlen, um eine charakteristische Lastkurve des Substrates zu ermitteln.

Das Vorgehen hat viele Vorteile. Durch dieses Verfahren wird es ermöglicht, eine charakteristische Lastkurve eines Substrates zu ermitteln und zu speichern. Eine solche Lastkurve kann in dem oben angeführten Verfahren als Soll-Lastkurve eingesetzt werden. Das ist sehr vorteilhaft, da auf diese Art und Weise grundsätzlich jederzeit eine neue Soll-Lastkurve ermittelt und abgespeichert werden kann. Es ist nicht nötig, eine Vielzahl von Parametern und unterschiedlichen Stoffen theoretisch zu berechnen und deren Einfluss empirisch oder halbempirisch zu berücksichtigen, sondern es kann einfach das aktuelle Substrat genommen werden und für den weiteren Betrieb kann sich die daraus ergebende Lastkurve als Soll-Lastkurve vorgegeben werden. Dadurch werden automatisch z.B. regionale Unterschiede bei der Zusammensetzung und Beschaffenheit von organischen Stoffen berücksichtigt. Außerdem wird die aktuelle Zusammensetzung des Substrats als Grundlage genommen.

Auf diese Art und Weise kann der Hersteller oder der Betreiber grundsätzlich jederzeit eine neue Soll-Lastkurve ermitteln.

Vorzugsweise werden in Abhängigkeit von der ermittelten charakteristischen Lastkurve der Betrieb der Rühreinrichtung und/oder eine Zufuhr von Gärstoffen und/oder Hilfsstoffen verändert.

In bevorzugten Weiterbildungen wird die ermittelte charakteristische Lastkurve mit einer Soll-Lastkurve verglichen. Bei dieser Betriebsart kann aufgrund eines Vergleichs der ermittelten charakteristischen Lastkurve mit einer Soll-Lastkurve auf die Eigenschaften des derzeitigen Substrates und dessen Verteilung innerhalb des Fermenters zurückgeschlossen werden. Wird die Lastkurve beispielsweise in einem oberen Bereich des Fermenters aufgenommen, kann auf eine Schwimmschicht zurückgeschlossen werden. Wird die Lastkurve in einem unteren Bereich des Fermenters aufgenommen wird der untere Bereich des Substrates durch die ermittelte Lastkurve charakterisiert.

Es ist bevorzugt, die charakteristische Lastkurve des Substrats periodisch zu ermitteln. Dabei kann ein zeitlicher Abstand zwischen der Ermittlung zweier charakteristischer Lastkurven variabel sein. Möglich ist es auch, einen konstanten zeitlichen Abstand zwischen der Ermittlung zweier Lastkurven vorzusehen. Möglich und bevorzugt ist es aber insbesondere, den zeitlichen Abstand der Ermittlung zweier charakteristischer Lastkurven auch von dem Ergebnis eines Vergleichs der charakteristischen Lastkurve mit einer Soll-Lastkurve bzw. Substrat-Lastkurve abhängig zu machen.

Besonders bevorzugt wird die Rühreinrichtung in der Höhe verfahren, um auf wenigstens zwei Höhenstellungen jeweils eine charakteristische Lastkurve des Substrates zu ermitteln. Dabei können Inhomogenitäten über der Höhe des Fermenters zuverlässig ermittelt werden.

So kann aus den ermittelten charakteristischen Lastkurven auf wenigstens zwei unterschiedlichen Höhen auf wenigstens eine Eigenschaft des Substrats zurückgeschlossen werden. Beispielsweise kann das Ergebnis sein, dass eine Entmischung stattgefunden hat und eine vermehrte oder verstärkte Umrührung erforderlich ist.

Demzufolge wird vorzugsweise in Abhängigkeit von den ermittelten charakteristischen Lastkurven auf wenigstens zwei unterschiedlichen Höhen gerührt, um das Substrat gezielt zu beeinflussen und insbesondere örtlich zu homogenisieren oder eine Verteilung von Substratanteilen zu verändern.

Möglich und bevorzugt ist ein gezieltes Gasaustreiben, wobei eine im Wesentlichen spiralförmige Ausrichtung eines Substratstromes durch die Rühreinrichtungen eingestellt wird. Dabei erfolgt das Austreiben vorzugsweise von unten nach oben.

Weitere Vorteile und Anwendungsmöglichkeiten der vorliegenden Erfindung ergeben sich aus dem Ausführungsbeispiel, welches im Folgenden mit Bezug auf die beiliegenden Figuren erläutert wird.

In den Figuren zeigen:
- Figur 1: einen schematischen Seitenquerschnitt durch einen Fermenter;
- Figur 2: eine perspektivische Ansicht einer Rühreinrichtung für den Fermenter gemäß Figur 1;
- Figur 3: eine geschnittene schematische Seitenansicht der Rühreinrichtung nach Figur 2;
- Figur 4: eine geschnittene schematische Seitenansicht der Rühreinrichtung nach Figur 3 ohne die Rührflügel;
- Figur 5: den Antriebsmotor der Rühreinrichtung nach Figur 4 im Schnitt;
- Figur 6: eine Vorderansicht der Rühreinrichtung gemäß Figur 5;

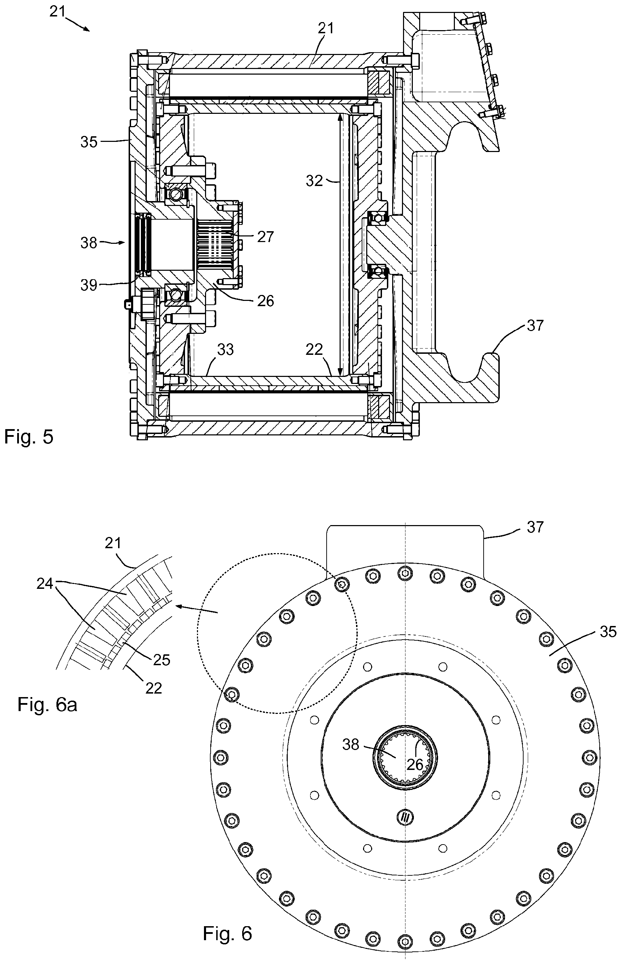

- Figur 6: ein vergrößertes Querschnittsdetail aus Figur 6;

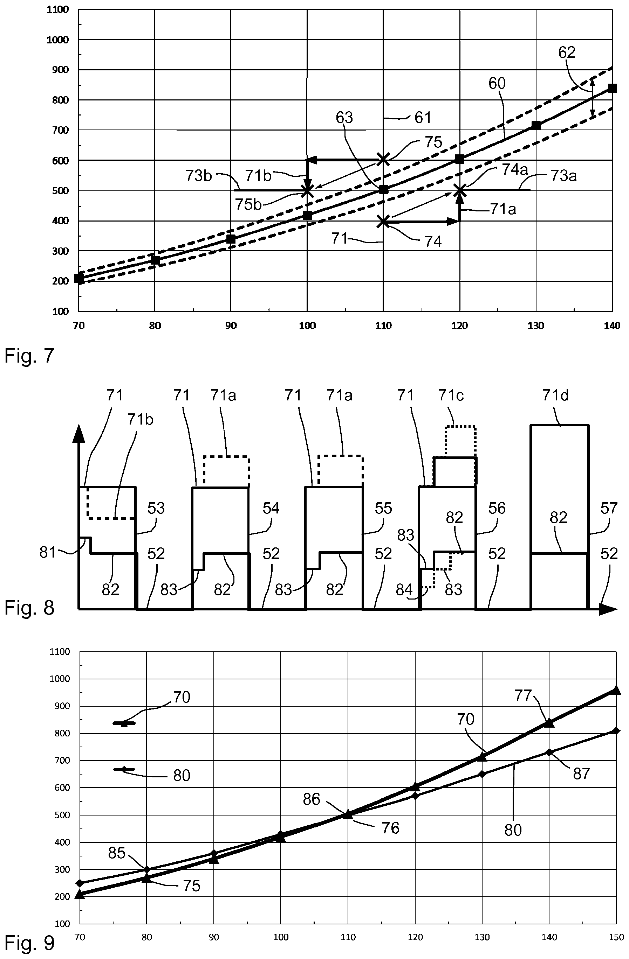

- Figur 7: eine Soll-Lastkurve für ein bestimmtes Substrat;
- Figur 8: die Drehzahl der Rühreinrichtung über der Zeit;
- Figur 9: eine Darstellung zweier aufgenommener unterschiedlicher Lastkurven.

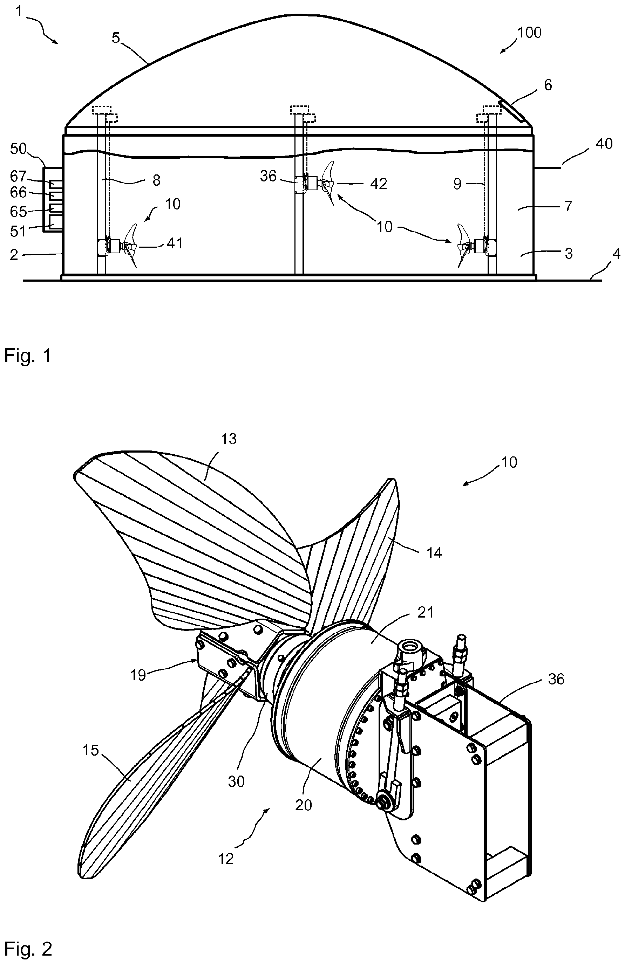

Mit Bezug auf die Figuren wird nun ein Ausführungsbeispiel erläutert. Dabei zeigt Figur 1 in einer stark schematischen Seitenansicht einen Fermenter 1 einer Biogasanlage 100.

Der Fermenter 1 weist vorzugsweise einen etwa kreisförmigen Querschnitt auf und verfügt über eine hier ringsum umlaufende Fermenterwandung 2 aus zum Beispiel Beton oder Stahl. Das Fermenterdach 5 kann ebenso wie der Boden als flaches Stahl- oder Betondach ausgeführt sein. Hier wird das Fermenterdach 5 durch ein insbesondere flexibles Material gebildet und erstreckt sich von der Wandung aus nach oben, sodass sich eine gewölbte Struktur des Behälterdaches 5 ergibt. Der Neigungswinkel des Fermenterdaches 5 hängt von den konkreten Bedingungen ab und kann 15° oder mehr und insbesondere auch 30° oder 45° erreichen oder überschreiten. Vorzugsweise ist das Fermenterdach 5 wenigstens teilweise und insbesondere vollständig entfernbar, um den Fermenterinnenraum 3 zugänglich zu machen. In dem Fermenterinnenraum 3 ist im Betrieb ein Substrat 7 vorgesehen.

In dem Fermenterdach 5 kann wenigstens eine Serviceöffnung 6 vorgesehen sein, um beispielsweise ein in dem Fermenterinnenraum 3 angeordnetes Rührgerät 10 zu warten. Eine Plattform 40 kann kann beispielsweise an der Außenseite der Fermenterwandung 2 befestigt sein, damit eine Bedienperson sich darauf stellen kann.

Figur 2 zeigt eine eine schematische perspektivische Darstellung der Rühreinrichtung 10 mit der Antriebseinrichtung 12. Die Rühreinrichtung 10 wird mittels einer Konsole 36 an der als Stützstange ausgebildeten Stützeinheit 8 höhenverstellbar aufgenommen. Das Rührgerät 10 ist zusammen mit der Stützeinheit 8 verschwenkbar und kann um 360° verdreht werden. Dadurch wird es ermöglicht, die Rührflügel 13, 14 und 15 zur Fermenterwandung 2 hin zu verschwenken und für Wartungszwecke nach oben zu verfahren, wo die Rühreinrichtung dann durch die Serviceöffnung 6 zugänglich ist.

Wie in Figur 1 dargestellt, können zwei, drei oder noch mehr Rühreinrichtungen 10 in dem Fermenterinnenraum 3 angeordnet werden, um für eine zuverlässige und ausreichende Durchmischung des Substrates 7 zu sorgen. Dabei ist es möglich, die verschiedenen Rühreinrichtungen 10 auf unterschiedlichen Höhen 41, 42 zu positionieren, um auf der Höhenposition 41 beispielsweise im unteren Bereich des Fermenters 1 umzurühren, während an der Höhenposition 42 ein oberer Bereich durchmischt wird, um Schwimmschichten aufzulösen bzw. zu vermeiden.

Neben den eingezeichneten Höhenstellungen 41 und 42 sind noch weitere Höhenstellungen möglich, insbesondere eine mittlere Höhenposition zwischen der ersten Höhenposition 41 und der zweiten Höhenstellung 42.

Vorzugsweise sind wenigstens zwei Rühreinrichtungen 10 vorgesehen, die jeweils um die Achse der Stützeinheit 8 verschwenkbar sind, um unterschiedliche Durchmischungen und Strömungsrichtungen innerhalb des Substrates 7 erzeugen zu können. Dabei können die Rühreinrichtungen 10 in die gleiche Umlaufrichtung oder schräg zueinander oder in entgegensetzte Umlaufrichtungen ausgerichtet sein. Der Einsatz ist auf der gleichen Höhe oder in unterschiedlichen Höhenpositionen möglich. Jede Rühreinrichtung 10 wird entweder durch eine eigene Steuereinrichtung 50 oder durch eine den Rührgeräten bzw. Rühreinrichtungen 10 gemeinsame Steuereinrichtung 50 gesteuert. Die Ansteuerung erfolgt hier über einen Frequenzumrichter 51.

Wie Figur 2 zeigt, weist eine Rühreinrichtung hier im Ausführungsbeispiel drei Rührflügel 13, 14 und 15 auf, die an einer Flügelnabe 19 angebracht sind. Die Flügelnabe 19 ist wiederum an der in Figur 2 nicht sichtbaren Antriebswelle drehfest befestigt.

Die Antriebseinrichtung 12 umfasst den Antriebsmotor 20 und die Vorsatzeinrichtung 30, die an dem Gehäuse des Antriebsmotors 20 befestigt ist. Der Antriebsmotor 20 weist einen großen Durchmesser auf, der durch den Außendurchmesser des Stators 21 wesentlich bestimmt wird. Der Stator 21 bildet mit seiner Außenseite einen Teil des Gehäuses des Antriebsmotors 20.

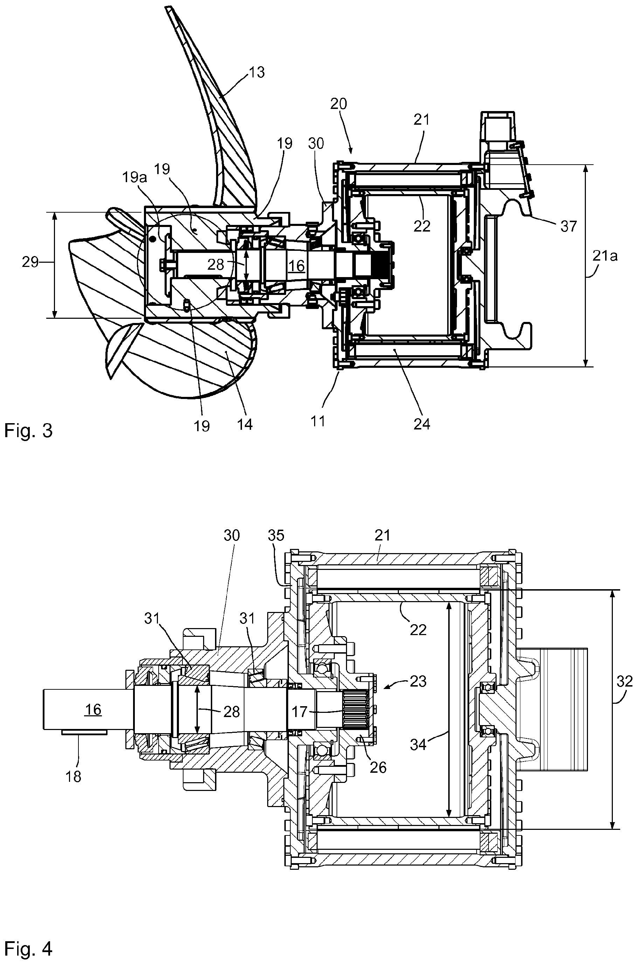

Figur 3 zeigt eine geschnittene schematische Seitenansicht der Rühreinrichtung 10, wobei hier die hinteren Rührflügel 13 und 14 zu sehen sind.

Erkennbar ist, dass an dem Antriebsmotor 20 eine Vorsatzeinrichtung 30 angebracht ist. Die Vorsatzeinrichtung dient zur Lagerung und Führung der Antriebswelle 16. Auf der Antriebswelle 16 ist die Flügelnabe 19 befestigt, an der wiederum die einzelnen Rührflügel 13 bis 15 befestigt sind. Die Konsolenaufnahme 37 dient zur Befestigung an der Konsole 36. Das Gehäuse 11 wird zum Teil durch den Stator 21 gebildet, der einen Außendurchmesser 21a aufweist. Im Inneren des Stators 21 ist hier ein hohl ausgebildeter Rotor 22 angeordnet. Der Stator 21 weist einen Außendurchmesser 21a auf. Die Antriebswelle 16 weist einen Außendurchmesser 28 auf. Der Außendurchmesser 21a des Stators ist mehrfach größer als der Außendurchmesser 28 der Antriebswelle 16. Dadurch wird ein besonders starkes Drehmoment des Antriebsmotors 20 erzielt. Hier ist auch ein Außendurchmesser 29 der Flügelnabe 19 erheblich kleiner als ein Außendurchmesser 21a des Stators.

Figur 4 zeigt einen detaillierteren Querschnitt des Antriebsmotors 20 mit der daran anmontierten Vorsatzeinrichtung 30. Die Vorsatzeinrichtung 30 ist an dem Stirndeckel 35 des Gehäuses 11 des Antriebsmotors 20 angebracht. In dem Inneren der Vorsatzeinrichtung 30 sind Lagereinrichtungen 31 zur Lagerung der Antriebswelle 16 angeordnet. Die Antriebswelle 16 verfügt über einen Mitnehmer 18, der radial nach außen absteht und in eine entsprechende Nut oder dergleichen in der Flügelnabe eintaucht.

Die Kopplung der Antriebswelle 16 mit dem Rotor 22 erfolgt über eine Kopplungseinrichtung 23, die hier als Zahnflansch 26 ausgebildet ist und die den Rotor 22 nach außen hin abdichtet. Der Zahnflansch 26 weist eine Innenverzahnung 27 auf, die im montierten Zustand, wie in Figur 4 dargestellt, im Eingriff mit der Außenverzahnung 17 der Antriebswelle 16 steht. Durch die Vorsatzeinrichtung 30 wird eine einfache und leichte Montage und ein einfacher Austausch ermöglicht. Im Bedarfsfalle wird die Vorsatzeinrichtung zusammen mit der Antriebswelle der Antriebseinrichtung 12 entnommen und kann durch eine neue ausgetauscht werden.

In Figur 4 ist der Innendurchmesser 32 des Rotors 22 21 eingezeichnet, Der Außendurchmesser 34 des Rotors 22 entspricht dem Innendurchmesser des Stators 21.

Der Innendurchmesser 32 beträgt ein Vielfaches des Außendurchmessers 28 der Antriebswelle 16, sodass von dem Antriebsmotor 20 hohe Drehmomente übertragbar sind.

Figur 5 zeigt den Antriebsmotor der Rühreinrichtung ohne Vorsatzeinrichtung im Schnitt. Dabei ist an der Rückseite die Konsolenaufnahme 37 und an der Vorderseite der Stirndeckel 35 zu sehen. In dem Stirndeckel 35 ist eine Wellenöffnung 38 zur Aufnahme der Antriebswelle 16 vorgesehen. An der Wellenöffnung 38 ist wenigstens eine Wellendichtung 39 angeordnet, um den Antriebsmotor nach innen hin abzudichten.

Nach dem Einführen der Antriebswelle 16 in die Wellenöffnung 38 greift die Außenverzahnung 17 der Antriebswelle 16 in die Innenverzahnung 27 des Zahnflansch 26 der Kopplungseinrichtung 23 ein. Es liegt eine drehfeste Kopplung zwischen der Antriebswelle und dem Antriebsmotor 20 vor. Durch den Aufbau mit einem hohlen Rotor 22 mit einem großen Innendurchmesser 32 wird ein leichter Aufbau mit einer hohen Drehmomentübertragbarkeit ermöglicht. Außerdem kann die Antriebswelle 16 ohne Öffnung des Antriebsmotors 20 ausgetauscht werden.

Figuren 6 und 6a zeigen eine Frontansicht bzw. eine vergrößerte geschnittene Frontansicht des Antriebsmotors 20 ohne Vorsatzeinrichtung 30. Im Hintergrund ist die Konsolenaufnahme 37 zu sehen, während vorn der Stirndeckel 35 mit der darin vorgesehenen Wellenöffnung 38 zu erkennen ist. Erkennbar ist der Zahnflansch 26 mit der Innenverzahnung 27.

Figur 6a zeigt eine vergrößerte geschnittene Darstellung eines Teils des Antriebsmotors 20, in der ein Winkelsegment des Stators 21 und des Rotors 22 erkennbar sind. An dem Stator 21 sind mehrere Wicklungen 24 vorgesehen, während an dem Rotor 22 Permanentmagnete 25 angeordnet sind. Vorzugsweise ist die Zahl der Wicklungen größer als die Zahl der Permanentmagnete und besonders bevorzugt beträgt die Anzahl der Permanentmagnete und der Wicklungen jeweils größer 30 und besonders bevorzugt größer 50. Durch die hohe Anzahl von Wicklungen und Permanentmagneten wird eine genaue Steuerung und es wird ein hohes Drehmoment ermöglicht. In bevorzugten Ausgestaltungen ist der Antriebsmotor 20 als Torquemotor ausgeführt. In einer bevorzugten Ausgestaltung sind z.B. mindestens 70 Pole, 35 Polpaare und/oder 280 Magnete vorgesehen.

Figur 7 zeigt eine Soll-Lastkurve 60 für ein bestimmtes Substrat 7 und einen sich um die Soll-Lastkurve 60 prozentual herum erstreckenden Toleranzbereich 62. Möglich ist es, dass sich der Toleranzbereich 62 um einen festen Wert um die Soll-Lastkurve herum erstreckt. Möglich ist auch ein relativer Prozentsatz von zum Beispiel 5 % oder 10 % Abweichung nach oben und nach unten.

In Figur 7 ist das Drehmoment in Newtonmetern über der Drehzahl in Umdrehungen pro Minute für einen konkreten Fall aufgetragen. Diese Soll-Lastkurve kann empirisch ermittelt werden und beispielsweise für ein bestimmtes Substrat mit einer bestimmten Zusammensetzung etc. gelten. Diese Soll-Lastkurve 60 wird vorgegeben, um über die Steuereinrichtung 50 die Rühreinrichtung 10 bzw. die Rühreinrichtungen 10 entsprechend gezielt steuern zu können.

Wie hier erkennbar, steigt das Drehmoment mit steigender Drehzahl an.

Grundsätzlich funktioniert die Steuerung bei dem Fermenter 1 derart, dass zunächst bei Start der Anlage eine Soll-Lastkurve 60 vorgegeben oder aus einer Speichereinrichtung abgerufen wird. Im Anschluss wird eine Soll-Drehzahl 61 durch die Steuereinrichtung 50 vorgegeben. Die Steuereinrichtung 50 betreibt die Rühreinrichtung 10 mit einer Ist-Drehzahl, die der vorgegebenen Soll-Drehzahl wenigstens etwa entspricht. Nach Erreichen der Ist-Drehzahl 71 wird an dem Betriebspunkt 74,75 ein Ist-Messwert aufgenommen, der für das Drehmoment oder die Leistung der Rühreinrichtung 10 bei der Ist-Drehzahl 71 charakteristisch ist. Beispielsweise kann über einen Dehnungsmessstreifen oder dergleichen auf der Antriebswelle oder in oder an dem Rotor ein Messwert aufgenommen werden, der charakteristisch für das anliegende Drehmoment ist. Möglich und bevorzugt ist es aber auch, einen solchen Messwert 73a,73b direkt aus der elektrischen Leistungsaufnahme der Rühreinrichtung 10 abzuleiten. Der Messwert kann direkt als Kennwert verwendet werden oder der Kennwert wird aus dem Messwert berechnet.

Aus dem Messwert wird ein Ist-Kennwert 74,75 abgeleitet. Dieser Ist-Kennwert wird mit dem sich aus der Soll-Lastkurve 60 bei der vorgegebenen Soll-Drehzahl 61 ergebenden Soll-Kennwert 63 für das Substrat verglichen.

Stellt die Steuereinrichtung fest, dass das auftretende Drehmoment bei der Ist-Drehzahl 71 außerhalb des Toleranzbereichs 62 bei der Soll-Drehzahl 61 liegt, so wird entweder die Ist-Drehzahl um ein vorbestimmtes Maß erhöht oder aber abgesenkt, je nachdem, ob das Ist-Drehmoment größer oder kleiner als der Soll-Kennwert 63 ist.

Im dargestellten Ausführungsbeispiel wird die Ist-Drehzahl 71 jeweils um 10 Umdrehungen/min erhöht oder reduziert. Möglich ist es aber auch, dass die Drehzahl in kleineren Schritten oder aber prozentual in Abhängigkeit von der Soll-Drehzahl 61 verändert wird.

Nach der Erhöhung der Ist-Drehzahl auf den Wert 71a steigt das steigt der Ist-Messwert und somit der Ist-Kennwert auf den Wert 73a, der hier im gewählten Ausführungsbeispiel innerhalb des Toleranzbereichs 62 um die Soll-Lastkurve 60 bei der Soll-Drehzahl 61 liegt. Durch die Erhöhung der Drehzahl hat sich das Drehmoment soweit erhöht, dass das Drehmoment nun im gewünschten Bereich liegt.

Tritt der umgekehrte Fall auf, d.h., dass das bei der Soll-Drehzahl 61 ein Drehmoment anliegt, welches oberhalb des Toleranzbereichs 62 der Soll-Lastkurve 60 liegt, so wird die Ist-Drehzahl 71 auf die Ist-Drehzahl 71b reduziert. Durch die geringere Drehzahl bedingt, nimmt auch das erforderliche Drehmoment ab, so dass der Ist-Kennwert 73b bei der verringerten Ist-Drehzahl 71b nun innerhalb des Toleranzbereichs 62 der Soll-Lastkurve 60 bei der Soll-Drehzahl 61 liegt.

Damit ist in beiden Fällen - also bei einer Überschreitung des Drehmomente nach oben und nach unten - jeweils dafür gesorgt, dass das Ist-Drehmoment sicher in dem gewünschten Bereich liegt. Im Anschluss wird für den Rest eines Rührzyklus die Rühreinrichtung mit der so ermittelten Ist-Drehzahl 71, 71a, oder 71b weiterbetrieben.

Falls die Erhöhung bzw. Absenkung des Ist-Drehmomente in einem Schritt nicht ausreichen sollte, wird die oben beschriebene Schleife iterativ durchgelaufen, bis das Ist-Drehmoment in dem gewünschten Zielbereich liegt.

Das bedeutet, dass bei dem Verfahrensablauf zunächst eine Soll-Lastkurve 60 in der Steuereinrichtung 50 hinterlegt wird bzw. aus einer Speichereinrichtung oder aus der Steuereinrichtung 50 eine Soll-Lastkurve 60 abgerufen wird.

Die Steuereinrichtung 50 gibt bei Beginn eines jeden Rührzyklus eine Soll-Drehzahl vor, zunächst die Soll-Drehzahl 61. Die Steuereinrichtung 50 steuert die Rühreinrichtung 10 entsprechend an, sodass die Rühreinrichtung 10 eine Ist-Drehzahl 71 erreicht, die im Rahmen der Regelgenauigkeit der vorgegebenen Soll-Drehzahl 61 entspricht. Es ergibt sich - je nach Eigenschaften des Substrates ein Betiebspunkt74 oder ein Betriebspunkt 75.

Anschließend erfasst die Steuereinrichtung einen Ist-Messwert 81 (vgl. Fig. 8), der für das Drehmoment der Rühreinrichtung 10 bei der Ist-Drehzahl 71 charakteristisch ist. Der Messwert 81 ist insbesondere die elektrische Leistungsaufnahme der Rühreinrichtung bei der Ist-Drehzahl 71,kann aber auch direkt das Drehmoment sein

Aus dem Ist-Messwert leitet die Steuereinrichtung 50 unter Berücksichtigung der Gerätefaktoren, der auftretenden Verluste etc. einen Ist-Kennwert für das aufgebrachte Drehmoment ab. Der Ist-Kennwert kann auch der abgegebenen Leistung bei der Ist-Drehzahl entsprechen, da sich aus der Leistung das Drehmoment bei bekannter Drehzahl berechnen lässt. Der Ist-Kennwert kann in einfachen Fällen dem Ist-Messwert entsprechen.

Die Steuereinrichtung 50 vergleicht im Anschluss den abgeleiteten Ist-Kennwert 81 mit dem sich aus der Soll-Lastkurve 60 bei der vorgegebenen Soll-Drehzahl 61 ergebenden Soll-Kennwert 63.

In Abhängigkeit von dem Ergebnis des Vergleichs steuert die Steuereinrichtung 50 die Rühreinrichtung 10.

Dabei ermittelt die Steuereinrichtung 50 insbesondere, ob der Ist-Kennwert innerhalb eines vorgegebenen Toleranzbereichs 62 um die Soll-Lastkurve 60 bei der Soll-Drehzahl 61 liegt.

Danach wird im Fall des Betriebspunktes 74, wenn nämlich der Ist-Kennwert unterhalb des Soll-Kennwerts 63 und außerhalb des Toleranzbereichs 62 liegt, die Ist-Drehzahl 71 der Rühreinrichtung 10 um ein vorbestimmtes Maß (hier 10 Umdrehungen/min) erhöht und es ergibt sich ein neuer Betriebspunkt 74a bei der neuen Ist-Drehzahl 71a mit einem Ist-Drehmoment 73a bzw. mit einem neuen Ist-Kennwert 73a.

Dann liegt der neue Betriebspunkt 74a innerhalb des vorgegebenen Toleranzbereichs 62 der Soll-Lastkurve 60 bei der Soll-Drehzahl 61 und der Rührzyklus wird mit dieser Drehzahl weiter betrieben.

Im Fall des Betriebspunktes 75, wenn nämlich der zugehörige Ist-Kennwert oberhalb des Soll-Kennwerts 63 und außerhalb des Toleranzbereichs 62 liegt, wird die Ist-Drehzahl 71 der Rühreinrichtung 10 um ein vorbestimmtes Maß (hier 10 Umdrehungen/min) verringert und es ergibt sich ein neuer Betriebspunkt 75b bei der neuen Ist-Drehzahl 71b mit einem Ist-Drehmoment 73b bzw. mit einem neuen Ist-Kennwert 73b.

Nun liegt auch der neue Betriebspunkt 75a innerhalb des vorgegebenen Toleranzbereichs 62 der Soll-Lastkurve 60 bei der Soll-Drehzahl 61 und der Rührzyklus wird mit dieser Drehzahl 71b weiter betrieben.

Figur 8 zeigt einen schematischen zeitlichen Steuerungsablauf zur Verdeutlichung des Prinzips. Aufgetragen sind die Drehzahl 71, 71a, 71b, 71c und 71d der Rühreinrichtung 10, die Messwerte 81 bis 84 und die aus der elektrischen Leistungsaufnahme resultierenden Kennwerte bzw. Drehmomente 91 bis 94 über der Zeit. Dargestellt sind mehrere Rührzyklen 53 bis 57, die von Rührpausen 52 unterbrochen werden.

Zu Beginn des Rührzyklus 53 wird die Rühreinrichtung 10 zunächst mit einer Ist-Drehzahl 71 angesteuert bzw. betrieben, die der Soll-Drehzahl 61 entspricht. Da die gemessene elektrische Leistung 81 bzw. das das resultierende Drehmoment 91 und somit der Kennwert zunächst über dem gewünschten Soll-Kennwert liegt, wird die Ist-Drehzahl auf den Wert 71b gesenkt, sodass die elektrische Leistungsaufnahme 82 sinkt und sich ein passendes Drehmoment bzw. Kennwert 92 ergibt, der nun im gewünschten Bereich liegt. Diese Drehzahl 71b wird dann bis zum Ende des Rührzyklus 53 beibehalten.

Der Kennwert 91 und der Messwert 81 (z. B. die Leistung) können linear miteinander verknüpft sein oder über eine sonstige Formel. Möglich ist es auch, dass als Kennwerte 91 bis 94 direkt die Messwerte 81 bis 84 verwendet werden, wenn die Zuordnung eindeutig und reproduzierbar ist.

Es schließt sich an den Rührzyklus 53 ein Ruhezyklus 52 an, in welchem hier die Drehzahl der Rühreinrichtung 10 auf null abgesenkt wird.

In dem folgenden Rührzyklus 54 wird wieder mit der Ist-Drehzahl 71 gestartet, die der Soll-Drehzahl 61 entspricht. Im Rührzyklus 54 liegt die elektrische Leistungsaufnahme 83 und somit der Kennwert bzw. das Drehmoment 93 zunächst unterhalb des Sollwertes, sodass die Drehzahl auf die Ist-Drehzahl 71a erhöht wird. Danach liegen die Leistungsaufnahme 82 und das Drehmoment 92 bzw. der Ist-Kennwert 92 im gewünschten Bereich. Hier in diesem Beispiel wird aus der Leistungsaufnahme mit der Drehzahl das Drehmoment berechnet.

Nach dem nächsten Ruhezyklus findet ein Rührzyklus 55 statt, der wiederum mit der Ist-Drehzahl 71 gestartet wird, die der Soll-Drehzahl 61 entspricht. Auch in diesem Rührzyklus wird zunächst wieder eine zu geringe Leistungsaufnahme 83 und somit ein zu geringes Drehmoment 93 detektiert, sodass die Drehzahl auf die Ist-Drehzahl 71a erhöht wird, bei der das gewünschte Ist-Drehmoment 92 anliegt.

Im nächsten Rührzyklus 56 kann ein identisches Verhalten vorliegen, wie durch die durchgezogen eingezeichnete Linie dargestellt ist. Möglich ist es aber auch, dass sich die Eigenschaften des Substrates 7 geändert haben und somit eine weitere Erhöhung der Ist-Drehzahl auf einen noch höheren Wert 71c erforderlich ist, wie gestrichelt dargestellt. Bei der gestrichelt dargestellten Variante im Rührzyklus 56 muss die Ist-Drehzahl in zwei Stufen auf die Werte 71a und 71c erhöht werden, bis das gewünschte Drehmoment erreicht wird. Dabei sind der Messwert 84 und der zugehörige Kennwert 94 zunächst zu gering, steigen dann auf den Messwert 83 bzw. den Kennwert 93 und erreichen erst bei Erhöhung der Drehzahl auf den Wert 71d den Messwert 82 und das gewünschte Drehmoment bzw. den Kennwert 92.

Jedes Mal, wenn in aufeinanderfolgenden Rührzyklen die Ist-Drehzahl erhöht werden muss wird ein erster Zähler 65 (vgl. Fig. 1) erhöht, sodass der Zähler beim vierten Rührzyklus 56 auf dem Wert 3 steht. Bei Überschreitung einer vorgegebenen Schwelle 67 von z. B. 3, 5 oder 10 oder dergleichen, so wird im sich daran anschließenden Rührzyklus 57 ein neuer Startwert für die Soll-Drehzahl vorgegeben. Der neue Sollwert ist dann direkt höher als der vorhergehende. Dies ist beispielhaft in Figur 8 im letzten Rührzyklus dargestellt, bei der eine Ist-Drehzahl 71d eingestellt wird.

Wird umgekehrt die Ist-Drehzahl verringert, wird ein zweiter Zähler 66 erhöht. Überschreitet der eine (gleiche oder andere) Schwelle 67, wird auch entsprechend reagiert.

Überschreitet der erste Zähler 65 oder der zweite Zähler 66 eine Schwelle 67, da in aufeinanderfolgenden Rührzyklen jeweils in dieselbe Richtung verschoben werden musste, werden insbesondere auch Handlungsmaßnahmen ausgegeben, wie z. B. weniger oder mehr Füttern (je nach Richtung), oder es wird eine andere Rührwerksposition angefahren oder es werden längere (oder kürzere) Rührzyklen durchgeführt,

Figur 9 zeigt zwei unterschiedliche Lastkurven 70 und 80, wobei jeweils das Drehmoment in Newtonmetern (Nm) über der Drehzahl in Umdrehungen pro Minute aufgetragen ist.

Die Lastkurven 70 und 80 repräsentieren hier zwei unterschiedliche Substrate 7, wobei die Lastkurve 70 mit dem Material "Schweinegülle" aufgenommen wurde und ein niedrigviskoses Medium darstellt. Die Lastkurve 80 wurde mit dem Material Gärrest eines Fermenters aufgenommen. Dieses Substrat für die Kurve 80 stellt ein mittelviskoses Medium dar.

Hier im Ausführungsbeispiel schneiden sich die beiden Lastkurven 70 und 80 bei den Messpunkten 76 und 86,während bei dem Messpunkt 75 das erforderliche Drehmoment der Lastkurve 70 geringer ist als das entsprechende Drehmoment an dem Messpunkt 85 der Lastkurve 80.

Während hier im Ausführungsbeispiel bei den dargestellten Lastkurven 70 und 80 das zur Drehung erforderliche Drehmoment bei geringen Drehzahlen (Messpunkte 75, 85) bei der Lastkurve 80 zunächst höher ist, wird das erforderliche Drehmoment zur Drehung der Rühreinrichtung 10 bei höheren Drehzahlen bei der Lastkurve 80 geringer als bei der Lastkurve 70.

Das bedeutet, dass mit dem Fermenter 1 und der darin angeordneten Rühreinrichtung 10 es möglich ist, Lastkurven 70, 80 des darin befindlichen Substrats 7 aufzunehmen. Anhand des Drehmomentverlaufs der Lastkurven 70 bzw. 80 können auf die jeweiligen Eigenschaften und gegebenenfalls die Zusammensetzung der jeweiligen Substrate 7 Rückschlüsse gezogen werden.

Beispielsweise kann die Lastkurve 70 die Soll-Lastkurve für das verwendete Substrat 7 darstellen. Wenn nun im Laufe des Betriebes eine Lastkurve mit der Rühreinrichtung und der Steuereinrichtung 50 aufgezeichnet wird und die aufgezeichnete Lastkurve der Lastkurve 80 entspricht, so können die Unterschiede der Lastkurven 70 und 80 ausgewertet werden und es können konkrete Handlungsempfehlungen ausgegeben oder direkt veranlasst werden, um die in dem Substrat vorhandene Lastkurve an die Soll-Lastkurve anzupassen. Es kann z.B. die Zusammensetzung des zugeführten Materials verändert werden. Es ist auch möglich, die Betriebsbedingungen der Rühreinrichtungen zu ändern und beispielsweise für eine bestimmte Zeit stärker zu rühren oder gegebenenfalls schwächer. Möglich ist es auch, die Gasabnahme in Abhängigkeit von den erfassten Lastkurven zu ändern

Möglich und bevorzugt ist es auch, die Rühreinrichtung 10 in Abhängigkeit von der gewünschten Gasabnahme zu steuern. Beispielsweise kann zu bestimmten Zeiten mehr Geld für abgelieferten Strom abgerechnet werden, sodass es empfehlenswert ist zu diesen Zeitpunkten mehr Gas und insbesondere Strom zu produzieren. Dadurch kann durch einen gezielten Einsatz der Rühreinrichtungen dafür gesorgt werden, dass zu diesen Zeitpunkten oder davor (zur Speicherung) eine erhöhte Gasausgabe erfolgt.

Mit den Rühreinrichtungen, die automatisch entlang der Höhe der Stützeinheiten 8 verfahrbar angeordnet sind, ist es auch möglich, auf unterschiedlichen Höhenstellungen 41,42 etc. Lastkurven 70, 80 des im Fermenterinnenraum sich befindenden Substrates 7 aufzunehmen. Über unterschiedliche Lastkurven 70, 80 in unterschiedlichen Höhen kann auf das Vorhandensein und die Größe von Schwimmschichten und weitere Parameter des Substrates zurückgeschlossen werden. Wird beispielsweise in bestimmten Höhenschichten eine geringe Viskosität detektiert, kann dies auf das Aufschwimmen bestimmter Bestandteile oder das Absenken anderer Bestandteile hindeuten. Durch entsprechende Messungen in Schichten darüber und darunter kann so auf eine inhomogene Verteilung in dem Substrat in dem Fermenter geschlossen werden.

Durch entsprechende Ansteuerung der Rühreinrichtungen 10 (Höhe, Winkel, Intensität) kann eine vollständigere Durchmischung erzielt werden.

Gezielte Strategien zum Gasaustreiben sind möglich, so z. B. eine spiralförmige automatische Anordnung, bei der das Austreiben von unten nach oben erfolgt
Durch die Aufzeichnung von Lastkurven 70, 80 ist es auch jederzeit möglich, die in der Steuereinrichtung 50 hinterlegte Soll-Lastkurve durch eine aktuell erfasste Lastkurve zu ersetzen. Wenn der Betreiber oder der Hersteller feststellt, dass sich der Fermenter 1 im derzeitigen Betrieb so verhält, wie erwünscht, kann eine neue Soll-Lastkurve 60 erstellt und abgespeichert werden. Das kann regelmäßig oder nur auf Wunsch geschehen, beispielsweise, wenn sich die Zusammensetzung des zugeführten Substrates ändert.

Insgesamt stellt die Erfindung eine Mediums-abhängige Rührwerkstechnik zur Verfügung, bei der in Abhängigkeit von den aktuellen Bedingungen des Substrats die Steuerung automatisch erfolgt.

Der Betrieb erfolgt Energieschonend. Durch die Steuerung wird das Substrat homogenisiert. Die Sollwerte ergeben sich je nach eingesetztem Medium. Der Zustand des Medium wird lokal erfasst.

Durch die Mess- und Steuerungswerte können Handlungsmaßnahmen ausgegeben werden. Es werden Abweichungen erfasst und Korrekturmaßnahmen vorgenommen oder vorgeschlagen. Bei Störungen werden Handlungsmaßnahmen vorgeschlagen. Insgesamt sind ein vollständiges Monitoring und eine Ferndiagnose möglich. Die Wartung kann vor Ort erfolgen.

Als Rührgerät wird ein hocheffizientes getriebeloses Rührwerk mit einem verlustarmen Direktantrieb eingesetzt, welches ein über der Drehzahl konstantes Drehmoment von bis zu 1000 Nm gewährleistet. Der Drehzahlbereich erstreckt sich stufenlos von 0 - 250 Umdrehungen pro Minute.

Der Leistungsbereich des im Ausführungsbeispiel beschriebenen Geräts beträgt 4 bis 12,5 kW. Der Volumenstrom beträgt hier bis zu 153 m3/min. Durch die komfortable Höhen- und Schwenkvorrichtung wird eine sichere Positionierung in Höhe und Winkel erreicht.

Zur Steuerung kann eine multifunktionale Kontrolle der Prozessdaten wie Volumenstrom, Druck, Drehmoment, Leistung, SET Kennzahlenkurve, Kennzahlenkurvenfunktion erfolgen.

### Bezugszeichenliste:

| | | | |
|---|---|---|---|
| 1 | Fermenter | 32 | Innendurchmesser 22 |
| 2 | Fermenterwandung | 33 | hohler Teil von 22 |
| 3 | Fermenterinnenraum | 34 | Außendurchmesser 22 |
| 4 | Horizontale | 35 | Stirndeckel |
| 5 | Fermenterdach | 36 | Konsole |
| 6 | Serviceöffnung | 37 | Konsolenaufnahme |
| 7 | Substrat | 38 | Wellenöffnung |
| 8 | Stützeinheit | 39 | Wellendichtung |
| 9 | Seil | 40 | Plattform |
| 10 | Rühreinrichtung, | 41 | 1. Höhenstellung |
| | Rührgerät | 42 | 2. Höhenstellung |
| 11 | Gehäuse | 50 | Steuereinrichtung |
| 12 | Antriebseinrichtung | 51 | Frequenzumrichter |
| 13-15 | Rührflügel | 52 | Ruhezyklus |
| 16 | Antriebswelle | 53-57 | Rührzyklus |
| 17 | Außenverzahnung | 60 | Soll-Lastkurve |
| 18 | Mitnehmer | 61 | Soll-Drehzahl |
| 19 | Flügelnabe | 62 | Toleranzbereich |
| 19a | Fixiereinheit | 63 | Soll-Kennwert |
| 20 | Antriebsmotor | 65 | erster Zähler |
| 21 | Stator | 66 | zweiter Zähler |
| 21a | Außendurchmesser 21 | 67 | Schwelle |
| 22 | Rotor | 70 | Lastkurve |
| 23 | Kopplungseinrichtung | 71 | Ist-Drehzahl |
| 24 | Wicklung | 71a,71b | Ist-Drehzahl |
| 25 | Permanentmagnet | 72a,72b | Ist-Messwert |
| 26 | Zahnflansch | 73a,73b | Ist-Kennwert |
| 27 | Innenverzahnung 26 | 74,74a | Betriebspunkt |
| 28 | Außendurchmesser 16 | 75,75a | Betriebspunkt |
| 29 | Außendurchmesser 19 | 81-84 | Messwert |
| 30 | Vorsatzeinrichtung | 91-94 | Kennwert |
| 31 | Lagereinrichtung 30 | 100 | Biogasanlage |

## Patentansprüche

1. Verfahren zum Herstellen von Biogas mit einem wenigstens teilweise mit Substrat (7) gefüllten Fermenter (1), wobei in dem Fermenter (1) wenigstens eine über eine Steuereinrichtung (50) gesteuerte Rühreinrichtung (10) angeordnet ist,
**gekennzeichnet durch** folgende Verfahrensschritte:
a) in der Steuereinrichtung (50) wird eine Soll-Lastkurve (60) hinterlegt;
b) die Steuereinrichtung (50) gibt eine Soll-Drehzahl (61) vor;
c) die Steuereinrichtung (50) betreibt die Rühreinrichtung (10) mit einer Ist-Drehzahl (71), die der vorgegebenen Soll-Drehzahl (61) entspricht;
d) die Steuereinrichtung erfasst einen Ist-Messwert (81), der für das Drehmoment der Rühreinrichtung (10) bei der Ist-Drehzahl (71) charakteristisch ist;
e) die Steuereinrichtung (50) leitet aus dem Ist-Messwert (81) einen Ist-Kennwert (91) für das aufgebrachte Drehmoment der Rühreinrichtung (10) ab;
f) die Steuereinrichtung (50) vergleicht den abgeleiteten Ist-Kennwert (91) mit dem sich aus der Soll-Lastkurve (60) bei der vorgegebenen Soll-Drehzahl (61) ergebenden Soll-Kennwert (63) für das Substrat (7);
g) die Steuereinrichtung (50) steuert die Rühreinrichtung (10) in Abhängigkeit von dem Ergebnis des Vergleiches.

2. Verfahren nach Anspruch 1, wobei die Steuereinrichtung (50) in einem Verfahrensschritt h) ermittelt, ob der Ist-Kennwert (73) innerhalb eines vorgegebenen Toleranzbereiches (62) um die Soll-Lastkurve (60) bei der Soll-Drehzahl (61) liegt.

3. Verfahren nach Anspruch 2, wobei die folgenden Verfahrensschritte i1) bis i4) in einer Schleife durchgeführt werden, falls der Ist-Kennwert (93) unterhalb des Soll-Kennwerts (63) und außerhalb des Toleranzbereichs (62) liegt:
i1) die Ist-Drehzahl (71) der Rühreinrichtung (10) wird um ein vorbestimmtes Maß erhöht,
i2) es wird ein neuer Ist-Messwert (82) bei der neuen Ist-Drehzahl (71a) erfasst,
i3) es wird aus dem neuen Ist-Messwert (82) einen neuer Ist-Kennwert (92) abgeleitet,
i4) die Schleife wird verlassen, wenn der abgeleitete neue Ist-Kennwert (92) innerhalb des vorgegebenen Toleranzbereichs (62) der Soll-Lastkurve (60) bei der Soll-Drehzahl (61) liegt,
oder wobei die folgenden Verfahrensschritte j1) bis j4) in einer Schleife durchgeführt werden, falls der Ist-Kennwert (91) oberhalb des Soll-Kennwerts (63) und außerhalb des Toleranzbereichs (62) liegt:
j1) die Ist-Drehzahl (71) der Rühreinrichtung (10) wird um ein vorbestimmtes Maß verringert,
j2) es wird ein neuer Ist-Messwert (82) bei der neuen Ist-Drehzahl (71b) erfasst,
j3) es wird aus dem neuen Ist-Messwert (82) ein neuer Ist-Kennwert (92) abgeleitet,
j4) die Schleife wird verlassen, wenn der abgeleitete neue Ist-Kennwert (92) innerhalb des vorgegebenen Toleranzbereichs (62) der Soll-Lastkurve (60) bei der Soll-Drehzahl (61) liegt,
und/oder wobei in Abhängigkeit von dem Ergebnis des Verfahrensschrittes h) in einer Schleife die Verfahrensschritte i1) bis i4) und/oder die die Verfahrensschritte j1) bis j4) durchgeführt werden.

4. Verfahren nach einem der vorhergehenden Ansprüche, wobei die eingestellte Ist-Drehzahl (71) für den Rest eines Rührzyklus (53) beibehalten wird, wenn der Ist-Kennwert (92) innerhalb des vorgegebenen Toleranzbereichs (62) um die Soll-Lastkurve (60) bei der vorgegebenen Soll-Drehzahl (61) liegt und/oder wobei am Ende eines Rührzyklus (53) die Rühreinrichtung (10) abgeschaltet wird und während eines sich anschließenden Ruhezyklus (52) abgeschaltet bleibt oder mit erheblich verringerter Leistung betrieben wird,
und wobei insbesondere nach einem Ruhezyklus (52) ein neuer Rührzyklus (53) folgt.

5. Verfahren nach einem der vorhergehenden Ansprüche 1 bis 3 oder nach Anspruch 4, wobei bei Start eines neuen Rührzyklus (53) die Verfahrensschritte b), c), d), e), f), g) und h) vorgenommen werden.

6. Verfahren nach einem der vorhergehenden Ansprüche, wobei die vorgegebene Soll-Drehzahl (61) von dem Substrat (7) und/oder dessen Zusammensetzung und/oder der gewünschten Biogasproduktion abhängt.

7. Verfahren nach einem der vorhergehenden Ansprüche, wobei in jedem Rührzyklus (53, 53) eine Ist-Drehzahl (71) in Abhängigkeit von der Soll-Lastkurve (60) eingestellt wird und anschließend für den Rest des Rührzyklus (53) beibehalten wird,
und/oder wobei ein erster Zähler (55) inkrementiert wird, wenn die Ist-Drehzahl (71a) größer als die vorgegebene Soll-Drehzahl (61) ist
und/oder wobei ein zweiter Zähler (56) inkrementiert wird, wenn die Ist-Drehzahl (71b) kleiner als die vorgegebene Soll-Drehzahl (61) ist,
und wobei insbesondere die Zuführmenge von dem Substrat (7) verändert und/oder die Zusammensetzung des zugeführten Substrats (7) verändert und/oder Zusatzstoffe zugeführt werden, wenn der der erste und/oder der zweite Zähler (65, 66) eine vorgegebene Schwelle (67) überschreitet,
wobei die vorgegebene Schwelle (67) insbesondere auch von der Anzahl der Rührzyklen abhängt,
und/oder wobei bei einer vorbestimmten Anzahl von direkt aufeinanderfolgenden Änderungen des ersten und/oder zweiten Zählers (65, 66) die Zuführmenge von Substrat (7) von außen und/oder die Zusammensetzung des zugeführten Substrats (7) verändert und/oder Zusatzstoffe zugeführt werden.

8. Verfahren nach einem der vorhergehenden Ansprüche, wobei als Ist-Messwert (81) ein Maß für die elektrische Leistung der Rühreinrichtung (10) oder für ein Drehmoment der Rühreinrichtung (10) gemessen wird.

9. Verfahren nach einem der vorhergehenden Ansprüche, die Steuereinrichtung (50) die Rühreinrichtung (10) mit einer Mehrzahl von unterschiedlichen Drehzahlen (71, 71a, 71b) betreibt, und jeweils ein Maß für die erforderliche Leistung der Rühreinrichtung (10) bei den unterschiedlichen Drehzahlen (71, 71a, 71b) erfasst, um eine charakteristische Lastkurve (70) des Substrates (7) zu ermitteln.

10. Verfahren nach dem Anspruch 9, wobei in Abhängigkeit von der charakteristischen Lastkurve (70) der Betrieb der Rühreinrichtung (10) und/oder eine Zufuhr von Gärstoffen und/oder Hilfsstoffen verändert wird.

11. Verfahren nach mindestens einem der Ansprüche 9 oder 10, wobei die charakteristische Lastkurve (70) mit einer Soll-Lastkurve (60) verglichen wird.

12. Verfahren nach mindestens einem der Ansprüche 9 bis 11, wobei die charakteristische Lastkurve (70) des Substrates (7) periodisch ermittelt wird.

13. Verfahren nach mindestens einem der Ansprüche 9 bis 12, wobei ein zeitlicher Abstand zwischen der Ermittlung zweier charakteristischer Lastkurven variabel ist.

14. Verfahren nach mindestens einem der Ansprüche 9 bis 13, wobei die Rühreinrichtung (10) in der Höhe verfahren wird, um auf wenigstens zwei Höhenstellungen (41, 42) jeweils eine charakteristische Lastkurve des Substrates zu ermitteln,
und wobei insbesondere in Abhängigkeit von den ermittelten charakteristischen Lastkurven auf zwei unterschiedlichen Höhen (41, 42) auf wenigstens eine Eigenschaft des Substrates (7) wie beispielsweise eine Entmischung zurückgeschlossen wird,
und/oder wobei in Abhängigkeit von den ermittelten charakteristischen Lastkurven auf wenigstens zwei unterschiedlichen Höhen gerührt wird, um das Substrat (7) gezielt zu beeinflussen und insbesondere örtlich zu homogenisieren oder eine Verteilung von Substratanteilen zu verändern.

15. Verfahren nach mindestens einem der vorhergehenden Ansprüche, wobei ein gezieltes Gasaustreiben erfolgt, wobei eine im Wesentlichen spiralförmige Ausrichtung eines Substratstromes durch die Rühreinrichtungen eingestellt wird, wobei insbesondere das Austreiben von unten nach oben erfolgt.

## Claims

1. Method for producing biogas by means of a digester (1) which is at least partially filled with a substrate (7), wherein at least one agitating device (10) controlled by a control device (50) is disposed in the digester (1),
**characterized by** the following process steps:
a) a target load curve (60) is lodged in the control device (50) ;
b) the control device (50) prescribes a target speed of rotation (61);
c) the control device (50) operates the agitating device (10) at an actual speed of rotation (71) corresponding to the prescribed target speed of rotation (61);
d) the control device captures an actual measurement value (81) which is characteristic of the torque of the agitating device (10) at the actual speed of rotation (71);
e) the control device (50) derives from the actual measurement value (81) an actual characteristic value (91) of the applied torque of the agitating device (10);
f) the control device (50) compares the derived actual characteristic value (91) against the target characteristic value (63) of the substrate (7) resulting from the target load curve (60) at the prescribed target speed of rotation (61) ;
g) the control device (50) controls the agitating device (10) in dependence on the result of comparison.

2. The method according to claim 1, wherein the control device (50) determines in a process step h) whether the actual characteristic value (73) lies within a prescribed tolerance range (62) around the target load curve (60) at the target speed of rotation (61).

3. The method according to claim 2, wherein the following process steps i1) through i4) are carried out in a loop, if the actual characteristic value (93) lies beneath the target characteristic value (63) and outside the tolerance range (62):
i1) the actual speed of rotation (71) of the agitating device (10) is increased a predetermined amount,
i2) a new actual measurement value (82) at the new actual speed of rotation (71a) is captured,
i3) a new actual characteristic value (92) is derived from the new actual measurement value (82),
i4) the loop is exited when the derived new actual characteristic value (92) lies within the prescribed tolerance range (62) of the target load curve (60) at the target speed of rotation (61),
or wherein the following process steps j1) through j4) are carried out in a loop, if the actual characteristic value (91) lies above the target characteristic value (63) and outside the tolerance range (62):
j1) the actual speed of rotation (71) of the agitating device (10) is decreased a predetermined amount,
j2) a new actual measurement value (82) at the new actual speed of rotation (71b) is captured,
j3) a new actual characteristic value (92) is derived from the new actual measurement value (82),
j4) the loop is exited when the derived new actual characteristic value (92) lies within the prescribed tolerance range (62) of the target load curve (60) at the target speed of rotation (61),
and/or wherein in dependence on the result of the process step h), the process steps i1) through i4) and/or the process steps j1) through j4) are carried out in a loop.

4. The method according to any of the preceding claims, wherein the actual speed of rotation (71) is maintained as set for the remainder of an agitating cycle (53), if the actual characteristic value (92) lies within the prescribed tolerance range (62) around the target load curve (60) at the prescribed target speed of rotation (61), and/or wherein the agitating device (10) is switched off as an agitating cycle (53) ends and remains switched off or is operated at considerably reduced power during a subsequent rest cycle (52),
and wherein a new agitating cycle (53) follows in particular after a rest cycle (52).

5. The method according to any of the preceding claims 1 to 3 or according to claim 4, wherein when a new agitating cycle (53) starts, the process steps b), c), d), e), f), g), and h) are carried out.

6. The method according to any of the preceding claims, wherein the prescribed target speed of rotation (61) is dependent on the substrate (7) and/or its composition and/or the desired biogas production.

7. The method according to any of the preceding claims, wherein an actual speed of rotation (71) is set in every agitating cycle (53, 53) in dependence on the target load curve (60) and maintained thereafter for the remainder of the agitating cycle (53),
and/or wherein a first counter (55) is incremented when the actual speed of rotation (71a) is higher than the prescribed target speed of rotation (61),
and/or wherein a second counter (56) is incremented when the actual speed of rotation (71b) is slower than the prescribed target speed of rotation (61),
and wherein in particular the fed quantity of substrate (7) is modified and/or the composition of the fed substrate (7) is changed and/or additives are supplied when the first and/or the second counter (65, 66) exceeds a prescribed threshold (67), wherein the prescribed threshold (67) in particular also depends on the number of agitating cycles,
and/or wherein in the case of a predetermined quantity of immediately successive modifications of the first and/or second counter (65, 66) the quantity of fed substrate (7) is changed from outside and/or the composition of the fed substrate (7) is modified and/or additives are added.

8. The method according to any of the preceding claims, wherein the actual measurement value (81) used is an amount measured for the electric power of the agitating device (10) or for a torque of the agitating device (10).

9. The method according to any of the preceding claims, wherein the control device (50) operates the agitating device (10) at a plurality of different speeds of rotation (71, 71a, 71b), every time capturing an amount for the required power of the agitating device (10) at the different speeds of rotation (71, 71a, 71b) to determine a characteristic load curve (70) of the substrate (7) .

10. The method according to claim 9, wherein the operation of the agitating device (10) and/or the feeding of ferments and/or additives are modified in dependence on the characteristic load curve (70).

11. The method according to at least one of the claims 9 or 10, wherein the characteristic load curve (70) is compared against a target load curve (60).

12. The method according to at least one of the claims 9 to 11, wherein the characteristic load curve (70) of the substrate (7) is determined periodically.

13. The method according to at least one of the claims 9 to 12, wherein a time interval between determining two characteristic load curves is variable.

14. The method according to at least one of the claims 9 to 13,
wherein the agitating device (10) is displaced in height to determine a characteristic load curve of the substrate at least in two height positions (41, 42),
and wherein conclusions are drawn about at least one property of the substrate (7), such as demixing, in particular in dependence on the determined characteristic load curves at two different heights (41, 42),
and/or wherein stirring is done in dependence on the determined characteristic load curves at least at two different heights for controlled influence on the substrate (7) and in particular for local homogenisation or modified distribution of substrate portions.

15. The method according to at least one of the preceding claims, wherein controlled gas expulsion takes place, wherein a substrate flow through the agitating devices is substantially set in a helical pattern, wherein in particular expelling takes place from bottom to top.

## Revendications

1. Procédé de production de biogaz, doté d'un fermenteur (1) rempli au moins partiellement de substrat (7), au moins un dispositif mélangeur (10) commandé par un dispositif de commande (50) étant agencé dans ledit fermenteur (1) **caractérisé par** les étapes de procédés suivantes :
a) une courbe de charge de consigne (60) est consignée dans le dispositif de commande (50) ;
b) le dispositif de commande (50) prédéfinit un régime moteur de consigne (61) ;
c) le dispositif de commande (50) fait fonctionner le dispositif mélangeur (10) à un régime moteur réel (71) qui correspond au régime moteur de consigne (61) prédéfini ;
d) le dispositif de commande détecte une valeur de mesure réelle (81) qui est caractéristique du couple de rotation du dispositif mélangeur (10) au régime moteur réel (71) ;
e) le dispositif de commande (50) dérive de ladite valeur de mesure réelle (81) un paramètre réel (91) du couple de rotation appliqué du dispositif mélangeur (10) ;
f) le dispositif de commande (50) compare le paramètre réel (91) dérivé au paramètre de consigne (63) du substrat (7) résultant de la courbe de charge (60) au régime moteur de consigne prédéfini (61) ;
g) le dispositif de commande (50) commande le dispositif mélangeur (10) en fonction du résultat de ladite comparaison.

2. Procédé selon la revendication 1, le dispositif de commande (50) calculant au cours d'une étape de procédé h) si le paramètre réel (73) se trouve, audit régime moteur (61), au sein d'une zone de tolérance (62) donnée autour de la cour de consigne (60).

3. Procédé selon la revendication 2, les étapes de procédé suivantes il) à i4) étant réalisées en boucle si le paramètre réel (93) est inférieur au paramètre de consigne (63) et se trouve hors de la zone de tolérance (62) :
i1) le régime moteur réel (71) du dispositif mélangeur (10) est augmenté d'une valeur prédéterminée,
i2) une nouvelle valeur de mesure réelle (82) est détectée au nouveau régime moteur réel (71a),
i3) un nouveau paramètre réel (92) est dérivé de la nouvelle valeur de mesure réelle (82),
i4) on quitte la boucle lorsque le nouveau paramètre réel dérivé (92) se trouve au sein de la zone de tolérance (62) prédéterminée de la courbe de charge de consigne (60) au régime moteur de consigne (61),
ou les étapes de procédé suivantes j1) à j4) étant réalisées en boucle si le paramètre réel (91) est supérieur au paramètre de consigne (63) et se trouve hors de la zone de tolérance (62) :
j1) le régime moteur réel (71) du dispositif mélangeur (10) est diminué d'une mesure prédéterminée,
j2) une nouvelle valeur de mesure réelle (82) est détectée au nouveau régime moteur réel (71b),
j3) un nouveau paramètre réel (92) est dérivé de la nouvelle valeur de mesure réelle (82),
j4) on quitte la boucle si le nouveau paramètre réel dérivé (92) se trouve au sein de la zone de tolérance (62) prédéterminée de la courbe de charge de consigne (60) au régime moteur de consigne (61),
et/ou, en fonction du résultat de l'étape de procédé h) en boucle, les étapes de procédé i1) à i4) et/ou les étapes de procédé j1) à j4) sont exécutées.

4. Procédé selon l'une quelconque des revendications précédentes, le régime moteur réel (71) réglé étant maintenu pour le reste d'un cycle de mélange (53) si le paramètre réel (92) se trouve au sein de la zone de tolérance prédéfinie (62) autour de la courbe de charge de consigne (60) au régime moteur de consigne prédéfini (61), et/ou en fin d'un cycle de mélange (53), le dispositif mélangeur (10) étant mis hors circuit et restant éteint au cours d'un cycle de repos (52) suivant ou est en marche à puissance nettement réduite,
et un nouveau cycle de mélange (53) suivant notamment après un cycle de repos (52).

5. Procédé selon l'une quelconque des revendications 1 à 3 précédentes ou selon la revendication 4, étant procédé aux étapes de procédé b), c), d), e), f), g) et h) au démarrage d'un nouveau cycle de mélange (53).

6. Procédé selon l'une quelconque des revendications précédentes, le régime moteur de consigne prédéfini (61) dépendant du substrat (7) et/ou de sa composition et/ou de la production de biogaz souhaitée.

7. Procédé selon l'une quelconque des revendications précédentes, dans chaque cycle de mélange (53) un régime moteur réel (71) étant réglé en fonction de la courbe de charge de consigne (60) pour être ensuite maintenu pour le reste du cycle de mélange (53),
et/ou un premier compteur (55) étant incrémenté lorsque le régime moteur réel (71a) est supérieur au régime moteur de consigne (61) prédéfini
et/ou un deuxième compteur (56) étant incrémenté lorsque le régime moteur réel (71b) est inférieur au régime moteur de consigne (61) prédéfini,
et notamment la quantité introduite de substrat (7) étant modifiée et/ou la composition du substrat (7) introduit étant modifiée et/ou des matières supplémentaires étant ajoutées lorsque le premier et/ou le deuxième compteur (65, 66) dépasse un seuil prédéterminé (67),
ledit seuil prédéterminé (67) dépendant notamment aussi du nombre de cycles de mélange,
et/ou, pour un nombre prédéterminé de modifications directement consécutives du premier et/ou du deuxième compteur (65, 66), la quantité de substrat introduite (7) de l'extérieur et/ou la composition du substrat (7) introduit étant modifiées et/ou des matières supplémentaires étant introduites.

8. Procédé selon l'une quelconque des revendications précédentes, une mesure de la puissance électrique du dispositif mélangeur (10) ou d'un couple de rotation du dispositif mélangeur (10) étant prise en tant que valeur de mesure réelle (81).

9. Procédé selon l'une quelconque des revendications précédentes, le dispositif de commande (50) faisant fonctionner le dispositif mélangeur (10) à une pluralité de régimes moteur (71, 71a, 71b) différents et détectant respectivement une mesure de la puissance nécessaire du dispositif mélangeur (10) aux différents régimes moteur (71, 71a, 71b) pour calculer une courbe de charge (70) caractéristique du substrat (7).

10. Procédé selon la revendication 9, le fonctionnement du dispositif mélangeur (10) et/ou une introduction de ferments et/ou d'adjuvants étant modifiée en fonction de la courbe de charge caractéristique (70).

11. Procédé selon au moins l'une quelconque des revendications 9 ou 10, la courbe de charge caractéristique (70) étant comparée à une courbe de charge (60) de consigne.

12. Procédé selon au moins l'une quelconque des revendications 9 à 11, la courbe de charge caractéristique (70) du substrat (7) étant calculée à intervalles périodiques.

13. Procédé selon au moins l'une quelconque des revendications 9 à 12, un écart temporaire entre le calcul de deux courbes de charge caractéristique pouvant varier.

14. Procédé selon au moins l'une quelconque des revendications 9 à 13, le dispositif mélangeur (10) étant déplacé en hauteur pour calculer respectivement une courbe de charge du substrat à au moins deux niveaux (41, 42),
et au moins une propriété du substrat (7) telle qu'une ségrégation étant notamment déduite en fonction des courbes de charge caractéristiques calculées à deux niveaux différents (41, 42),
et/ou étant procédé au mélange en fonction des courbes de charge caractéristiques calculées à au moins deux niveaux différents, pour impacter le substrat (7) de manière ciblée et pour l'homogénéiser notamment localement ou modifier une répartition de portions de substrat.

15. Procédé selon au moins l'une quelconque des revendications précédentes, un dégazage ciblé ayant lieu, une direction en vrille du flux de substrat étant sensiblement réglée par les dispositifs mélangeurs, le dégazage s'effectuant notamment de bas en haut.
